(19) Europäisches Patentamt
European Patent Office
Office européen des brevets

(11) Veröffentlichungsnummer: **0 469 425 B1**

(12) **EUROPÄISCHE PATENTSCHRIFT**

(45) Veröffentlichungstag der Patentschrift: **04.01.95**

(51) Int. Cl.[6]: **C07D 333/54**, C07D 333/62, A01N 43/12

(21) Anmeldenummer: **91112221.6**

(22) Anmeldetag: **22.07.91**

# (54) 2,2'-Thienyl-benzothiophene, ihre Herstellung und ihre Verwendung zur Bekämpfung von Schädlingen.

(30) Priorität: **31.07.90 DE 4024206**

(43) Veröffentlichungstag der Anmeldung:
**05.02.92 Patentblatt 92/06**

(45) Bekanntmachung des Hinweises auf die Patenterteilung:
**04.01.95 Patentblatt 95/01**

(84) Benannte Vertragsstaaten:
**BE CH DE DK ES FR GB IT LI NL**

(56) Entgegenhaltungen:
**EP-A- 0 054 233**
**EP-A- 0 353 667**
**WO-A-86/05949**
**US-A- 3 706 767**

**TETRAHEDRON LETTERS. Bd. 46, 1972, OXFORDGB Seiten 4069 - 4072; K. J. BROWN ET AL.: 'A new approach to the synthesis of 2-substituted benzothiophenes and their hetero-analogues'**

(73) Patentinhaber: **BASF Aktiengesellschaft**
**Carl-Bosch-Strasse 38**
**D-67063 Ludwigshafen (DE)**

(72) Erfinder: **Kober, Reiner, Dr.**
**Im Schlittweg 20**
**W-6701 Fussgoenheim (DE)**
Erfinder: **Thoebald, Hans, Dr.**
**Oueichstrasse 6**
**W-6703 Limburgerhof (DE)**
Erfinder: **Kardorff, Uwe, Dr.**
**D 3,4**
**W-6800 Mannheim 1 (DE)**
Erfinder: **Kuenast, Christoph Dr.**
**Salierstrasse 2**
**W-6701 Otterstadt (DE)**
Erfinder: **Hofmeister, Peter, Dr.**
**Bernard-Humblot-Strasse 12**
**W-6730 Neustadt (DE)**
Erfinder: **Seele, Rainer, Dr.**
**Leiblstrasse 3**
**W-6701 Fussgoenheim (DE)**
Erfinder: **Wagenblast, Gerhard, Dr.**
**Bachweg 8**
**W-6719 Weisenheim (DE)**

**EP 0 469 425 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Vorliegende Erfindung betrifft 2,2'-Thienyl-benzothiophene der allgemeinen Formel I,

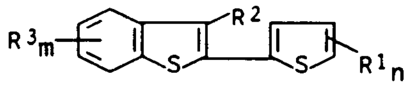

I

in der die Substituenten und die Indizes die folgende Bedeutung haben:

- $R^1$ und $R^3$ unabhängig voneinander Halogen, $C_1$-$C_8$-Alkyl, $C_1$-$C_6$-Halogenalkyl, $C_1$-$C_6$-Alkoxy und $C_1$-$C_6$-Halogenalkoxy,
- $R^2$ Wasserstoff, Halogen, $C_1$-$C_8$-Alkyl, $C_1$-$C_6$-Halogenalkyl, $C_1$-$C_6$-Alkoxy und $C_1$-$C_6$-Halogenalkoxy,
- n 0, 1 oder 2, wobei die Reste verschieden sein können, wenn n für 2 steht,
- m 0, 1, 2, 3 oder 4, wobei die Reste verschieden sein können, wenn m für 2, 3 oder 4 steht,

und wobei n und m nicht gleichzeitig 0 bedeuten, wenn $R^2$ Wasserstoff bedeutet.

Des weiteren betrifft die Erfindung verfahren zur Herstellung dieser Verbindungen, sie enthaltende Mittel und die Verwendung von Verbindungen der allgemeinen Formel IA

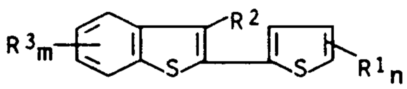

IA

in der die Substituenten und die Indizes die folgende Bedeutung haben:

- $R^1$ und $R^3$ unabhängig voneinander Halogen, $C_1$-$C_8$-Alkyl, $C_1$-$C_6$-Halogenalkyl, $C_1$-$C_6$-Alkoxy und $C_1$-$C_6$-Halogenalkoxy,
- $R^2$ Wasserstoff, Halogen, $C_1$-$C_8$-Alkyl, $C_1$-$C_6$-Halogenalkyl, $C_1$-$C_6$-Alkoxy und $C_1$-$C_6$-Halogenalkoxy,
- n 0, 1 oder 2, wobei die Reste verschieden sein können, wenn n für 2 steht,
- m 0, 1, 2, 3 oder 4, wobei die Reste verschieden sein können, wenn m für 2, 3 oder 4 steht,

zur Bekämpfung von Schädlingen.

Es ist bekannt, daß bestimmte Bis- und Terthiophenverbindungen nematodizid wirksam sind (US-A 3,050,442; WO-A 86/05949). Derartige Verbindungen sind außerdem als herbizid wirksam bekannt (EP-A 353 667). Neben den bei Schädlingsbekämpfungsmitteln unerwünschten herbiziden Eigenschaften führen die bekannten Bis- und Terthiophenverbindungen bei Hautkontakt zu für die Anwendung unerwünschten Irritationen (Photochem. Photobiol., 35, 615 f. (1982)).

Außerdem wird die Herstellung von 2,2'-Thienyl-benzothiophen beschrieben (Tetrahedron Letters, 46, 4069 (1974); J. Sci. Ind. Res., Sect. B 19, 395 und 400 (1960)). Ob es sich bei dem nach dieser Literatur beschriebenen Produkt jedoch tatsächlich um 2,2'-Thienyl-benzothiophen handelt ist fragwürdig, da der beschriebene Schmelzpunkt um mehr als 20 °C höher liegt als der Schmelzpunkt des im Rahmen der vorliegenden Erfindung erhaltenen 2,2'-Thienyl-benzothiophen, dessen Struktur aufgrund von Herstellungsmethoden mit eindeutiger Verknüpfungsreaktion (Wittig-Synthese ausgehend von Thiophen-2-carbonsäurechlorid und dem Triphenylphosphoniumsalz von 2-Mercaptobenzylbromid und anschließende Cyclisierung) und physikalischen Untersuchungen ($^1$H- bzw. $^{13}$C-NMR-, IR-Daten und Verbrennungsanalyse) gesichert ist. Versuche, 2,2'-Thienyl-benzothiophen nach den aus der Literatur bekannten Verfahren herzustellen, schlugen bislang fehl.

Der Erfindung lagen neue insektizid wirksame Verbindungen als Aufgabe zugrunde, welche die vorstehend geschilderten Nachteile bezüglich der herbiziden Wirkung und der Gefahr für den Anwender nicht zeigen.

Demgemäß wurden die eingangs definierten Verbindungen I gefunden.

Außerdem wurden Verfahren zur Herstellung der Verbindungen I sowie die Verwendung der Verbindungen IA zur Bekämpfung von Schädlingen gefunden.

Man erhält die Verbindungen I beispielsweise dadurch, daß man ein 2-Halogen-benzothiophen der allgemeinen Formel II in an sich bekannter Weise (Heterocycles, 24 (10), 2901 f. (1986)) in einem inerten organischen Lösungsmittel in Gegenwart eines Katalysators mit einer Thienyl-Grignard Verbindung der allgemeinen Formel III kuppelt.

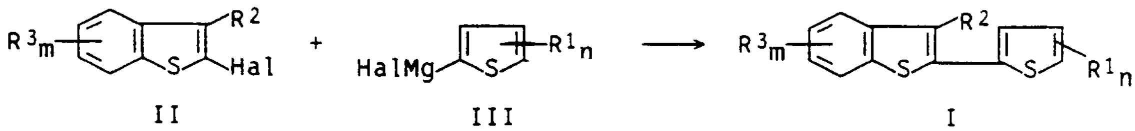

Hal in den Formeln II und III steht unabhängig voneinander für Halogen wie Fluor, Chlor, Brom und Jod, insbesondere für Chlor und Brom.

Als Lösungsmittel für diese Umsetzung dienen organische Lösungsmittel wie Ether wie insbesondere Diethylether, Diisobutylether, tert.-Butylmethylether, Tetrahydrofuran und Dioxan und aliphatische und aromatische, gegebenenfalls halogenierte Kohlenwasserstoffe wie insbesondere n-Pentan, n-Hexan, Cyclohexan, Toluol und Chlorbenzol sowie entsprechende Gemische.

Als Katalysator eignet sich insbesondere Ni (DPPP)$Cl_2$ (DPPP = 1,3-Bis(diphenylphosphino)propan). Der Katalysator wird im allgemeinen in bis zu 5,0 mol-%, vorzugsweise bis zu 2,0 mol-%, insbesondere von 1,1 bis 1,35 mol-%, bezogen auf das Edukt II bzw. III eingesetzt.

Die Umsetzung verläuft gewöhnlich oberhalb von 0 °C mit ausreichender Geschwindigkeit. Da die Reaktion meist exotherm verläuft, kann es von Vorteil sein, eine Kühlquelle vorzusehen.

In der Regel erfolgt die Umsetzung bei Temperaturen von 0 bis 50 °C, vorzugsweise 10 bis 40 °C, insbesondere 20 bis 30 °C.

Im allgemeinen werden die Verbindungen III und die Verbindungen II in äquimolaren Mengen miteinander umgesetzt. Es kann vorteilhaft für die Ausbeute sein, das Benzothiophen in einem über- oder Unterschuß bezogen auf das Grignard-Reagens einzusetzen. Üblicherweise wird man aus wirtschaftlicher Sicht dasjenige Edukt im überschuß verwenden, welches leichter herzustellen bzw. billiger zu erhalten ist.

Die für die Umsetzung benötigten 2-Halogenbenzothiophenderivate der allgemeinen Formel II sind aus der allgemeinen Literatur bekannt oder können entsprechend den dort beschriebenen Verfahren hergestellt werden (vgl. Adv. in Het. Chem., Vol. 11, Seite 370f (1970)).

Die für die Herstellung der Verbindungen I benötigten Thienyl-Grignard Verbindungen III sind aus der eingangs Zitierten Literatur bekannt oder nach den dort beschriebenen Verfahren zugänglich (vgl. EP-A 353 667; Heterocycles, Vol. 24 (10), 2902 f (1986)).

Im Hinblick auf die bestimmungsgemäße Verwendung der Verbindungen IA kommen als Substituenten folgende Reste in Betracht:

$R^1$ und $R^3$ unabhängig voneinander

Halogen wie Fluor, Chlor, Brom und Jod, insbesondere Chlor und Brom;

$C_1$-$C_8$-Alkyl, besonders verzweigtes oder unverzweigtes $C_1$-$C_6$-Alkyl wie Methyl, Ethyl, Propyl, 1-Methylethyl, Butyl, 1-Methyl-propyl, 2-Methyl-propyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethyl-butyl, 2-Ethylbutyl, 1,1,2-Trimethylpropyl, 1,2,2-Trimethylpropyl, 1-Ethyl-1-methylpropyl und 1-Ethyl-2-methylpropyl, insbesondere verzweigtes oder unverzweigtes $C_1$-$C_4$-Alkyl wie Methyl, Ethyl, Propyl, 1-Methylethyl, Butyl, 1-Methyl-propyl, 2-Methylpropyl und 1,1-Dimethylethyl;

$C_1$-$C_6$-Halogenalkyl, vorzugsweise $C_1$-$C_4$-Halogenalkyl, besonders $C_1$-$C_2$-Halogenalkyl wie Chlormethyl, Dichlormethyl, Trichlormethyl, Fluormethyl, Difluormethyll, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyi, 2,2,2-Trichlorethyl und Pentafluorethyl, insbesondere Trifluormethyl;

$C_1$-$C_6$-Alkoxy wie Methoxy, Ethoxy, Propyloxy, 1-Methylethoxy, Butyloxy, 1-Methyl-propyloxy, 2-Methylpropyloxy, 1,1-Dimethylethoxy, Pentyloxy, 1-Methylbutyloxy, 2-Methylbutyloxy, 3-Methylbutyloxy, 2,2-Di-methylpropyloxy, 1-Ethylpropyloxy, Hexyloxy, 1,1-Dimethylpropyloxy, 1,2-Dimethylpropyloxy, 1-Methylpentyloxy, 2-Methylpentyloxy, 3-Methylpentyloxy, 4-Methylpentyloxy, 1,1-Dimethylbutyloxy, 1,2-Dimethylbutyloxy, 1,3-Dimethylbutyloxy, 2,2-Dimethylbutyloxy, 2,3-Dimethylbutyloxy, 3,3-Dimethylbutyloxy, 1-Ethyl-butyloxy, 2-Ethylbutyloxy, 1,1,2-Trimethylpropyloxy, 1,2,2-Trimethylpropyloxy, 1-Ethyl-1-methylpropyloxy und 1-Ethyl-2-methylpropyloxy, insbesondere Methoxy, Ethoxy und 1-Methylethoxy;

und

$C_1$-$C_6$-Halogenalkoxy, vorzugsweise $C_1$-$C_4$-Halogenalkoxy, besonders $C_1$-$C_2$-Halogenalkoxy wie Chlormethyloxy, Dichlormethyloxy, Trichlormethyloxy, Chlorfluormethyloxy, Dichlorfluormethyloxy, Fluormethoxy, Difluormethoxy, Trifluormethoxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy,

2,2,2-Trichlorethyloxy und Pentafluorethyloxy, insbesondere Trifluormethoxy;
R2
Wasserstoff,
Halogen wie Fluor, Chlor, Brom und Jod, insbesondere Chlor und Brom;
$C_1$-$C_8$-Alkyl, besonders verzweigtes oder unverzweigtes $C_1$-$C_6$-Alkyl wie Methyl, Ethyl, Propyl, 1-Methylethyl, Butyl, 1-Methyl-propyl, 2-Methyl-propyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethyl-butyl, 2,3-Dimethylbutyl, 3,3-Dimethylbutyl, 1-Ethyl- butyl, 2-Ethylbutyl, 1,1,2-Trimethylpropyl, 1,2,2-Trimethylpropyl, 1-Ethyl-1-methylpropyl und 1-Ethyl-2-methylpropyl, insbesondere verzweigtes oder unverzweigtes $C_1$-$C_4$-Alkyl wie Methyl, Ethyl, Propyl, 1-Methylethyl, Butyl, 1-Methyl-propyl, 2-Methylpropyl und 1,1-Dimethylethyl;
$C_1$-$C_6$-Halogenalkyl, vorzugsweise $C_1$-$C_4$-Halogenalkyl, besonders $C_1$-$C_2$-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl, insbesondere Trifluormethyl;
$C_1$-$C_6$-Alkoxy wie Methoxy, Ethoxy, Propyloxy, 1-Methylethoxy, Butyloxy, 1-Methyl-propyloxy, 2-Methylpropyloxy, 1,1-Dimethylethyloxy, Pentyloxy, 1-Methylbutyloxy, 2-Methylbutyloxy, 3-Methylbutyloxy, 2,2-Dimethylpropyloxy, 1-Ethylpropyloxy, Hexyloxy, 1,1-Dimethylpropyloxy, 1,2-Dimethylpropyloxy, 1-Methylpentyloxy, 2-Methylpentyloxy, 3-Methylpentyloxy, 4-Methylpentyloxy, 1,1-Dimethylbutyloxy, 1,2-Dimethylbutyloxy, 1,3-Dimethylbutyloxy, 2,2-Dimethylbutyloxy, 2,3-Dimethylbutyloxy, 3,3-Dimethylbutyloxy, 1-Ethyl-butyloxy, 2-Ethylbutyloxy, 1,1,2-Trimethylpropyloxy, 1,2,2-Trimethylpropyloxy, 1-Ethyl-1-methylpropyloxy und 1-Ethyl-2-methylpropyloxy, insbesondere Methoxy;
$C_1$-$C_6$-Halogenalkoxy, vorzugsweise $C_1$-$C_4$-Halogenalkoxy, besonders $C_1$-$C_2$-Halogenalkoxy wie Chlormethyloxy, Dichlormethyloxy, Trichlormethyloxy, Fluormethoxy, Difluormethoxy, Trifluormethoxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy;
n
0, 1 oder 2, wobei die Reste verschieden sein können, wenn n für 2 steht,
m
0, 1, 2, 3 oder 4, wobei die Reste verschieden sein können, wenn m für 2, 3 oder 4 steht.
Besonders bevorzugt sind 2,2'-Thienyl-benzothiophene der allgemeinen Formel IA, in denen die Reste die folgende Bedeutung haben:
$R^1$ und $R^3$ unabhängig voneinander
Halogen wie Fluor, Chlor, Brom und Jod, insbesondere Chlor und Brom;
verzweigtes oder unverzweigtes $C_1$-$C_6$-Alkyl wie Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethyl-butyl, 2-Ethylbutyl, 1,1,2-Trimethylpropyl, 1,2,2-Trimethylpropyl, 1-Ethyl-1-methylpropyl und 1-Ethyl-2-methylpropyl, vorzugsweise verzweigtes oder unverzweigtes $C_1$-$C_4$-Alkyl wie Methyl, Ethyl, Propyl, 1-Methylethyl, Butyl, 1-Methyl-propyl, 2-Methylpropyl und 1,1-Dimethylethyl;
$C_1$-$C_4$-Halogenalkyl, besonders $C_1$-$C_2$-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
$C_1$-$C_4$-Alkoxy wie Methoxy, Ethoxy, Propyloxy, 1-Methylethoxy, Butyloxy, 1-Methyl-propyloxy, 2-Methylpropyloxy und 1,1-Dimethylethyloxy;
$C_1$-$C_4$-Halogenalkoxy, besonders $C_1$-$C_2$-Halogenalkoxy wie Chlormethyloxy, Dichlormethyloxy, Trichlormethyloxy, Fluormethoxy, Difluormethoxy, Trifluormethoxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy;
R2
Wasserstoff,

Halogen wie Fluor, Chlor, Brom und Jod, insbesondere Chlor und Brom;
verzweigtes oder unverzweigtes $C_1$-$C_6$-Alkyl wie Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, vorzugsweise verzweigtes oder unverzweigtes $C_1$-$C_4$-Alkyl wie Methyl, Ethyl, Propyl, 1-Methylethyl, Butyl, 1-Methyl-propyl, 2-Methylpropyl und 1,1-Dimethylethyl;
$C_1$-$C_4$-Halogenalkyl, besonders $C_1$-$C_2$-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
$C_1$-$C_4$-Alkoxy wie Methoxy, Ethoxy, Propyloxy, 1-Methylethoxy, Butyloxy, 1-Methyl-propyloxy, 2-Methylpropyloxy und 1,1-Dimethylethyloxy;
$C_1$-$C_4$-Halogenalkoxy, besonders $C_1$-$C_2$-Halogenalkoxy wie Chlormethyloxy, Dichlormethyloxy, Trichlormethyloxy, Fluormethoxy, Difluormethoxy, Trifluormethoxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy;
n
0, 1 oder 2, wobei die Reste verschieden sein können, wenn n für 2 steht,
m
0, 1, 2 oder 3, wobei die Reste verschieden sein können, wenn m für 2 oder 3 steht.

Insbesondere bevorzugt sind 2,2'-Thienyl-benzothiophene der allgemeinen Formel IA, in denen die Reste die folgende Bedeutung haben:
$R^1$ und $R^3$ unabhängig voneinander
Halogen wie Fluor, Chlor, Brom und Jod, insbesondere Chlor und Brom;
verzweigtes oder unverzweigtes $C_1$-$C_4$-Alkyl wie Methyl, Ethyl, Propyl, 1-Methylethyl, Butyl, 1-Methylpropyl, 2-Methylpropyl und 1,1-Dimethylethyl ;
$C_1$-$C_2$-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
$C_1$-$C_2$-Alkoxy wie Methoxy und Ethoxy;
$C_1$-$C_2$-Halogenalkoxy wie Chlormethyloxy, Dichlormethyloxy, Trichlormethyloxy, Fluormethoxy, Difluormethoxy, Trifluormethoxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy;
$R^2$
Wasserstoff,
Halogen wie Fluor, Chlor, Brom und Jod, insbesondere Chlor und Brom;
verzweigtes oder unverzweigtes $C_1$-$C_4$-Alkyl wie Methyl, Ethyl, Propyl, 1-Methylethyl, Butyl, 1-Methylpropyl, 2-Methylpropyl und 1,1-Dimethylethyl ;
$C_1$-$C_2$-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
$C_1$-$C_2$-Alkoxy wie Methoxy und Ethoxy;
$C_1$-$C_2$-Halogenalkoxy wie Chlormethyloxy, Dichlormethyloxy, Trichlormethyloxy, Fluormethoxy, Difluormethoxy, Trifluormethoxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy;
n 0 oder 1,
m 0, 1 oder 2, wobei die Reste verschieden sein können, wenn m für 2 steht.

Beispiele für insbesondere bevorzugte Verbindungen der allgemeinen Formel IA sind in der folgenden Tabelle aufgeführt.

Tabelle

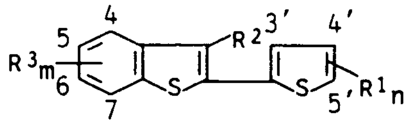

IA

| $(R^1)$ | $R^2$ | $(R^3)_m$ |
|---|---|---|
| - | H | - |
| 5'-Cl | H | - |
| 5'-Br | H | - |
| 5'-$CH_3$ | H | - |
| 5'-Cl | H | 6-$CH_3O$ |
| 5'-$CH_3$ | H | 6-$CF_3$ |
| 5'-Cl | H | 5-Cl |
| 5'-$CH_3$ | Cl | 5-Cl |
| 5'-$OCH_3$ | H | 5-Cl |
| 5'-Cl | H | 7-$CH_3$ |
| 4'-$CH_3$ | Cl | 7-$CH_3$ |
| 5'-$CH_2CH_3$ | H | 5-$CF_3$ |
| 5'-Br | Br | - |
| - | Br | - |

Die Verbindungen der Formel IA sind geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinär-Sektor als Schädlingsbekämpfungsmittel eingesetzt werden.

Zu den schädlichen Insekten gehören aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grndiosella, Earias insulana, Elasmopalpus lignosellus, Eupoecilia ambiguella, Evetria bouliana, Feltia subterranea, Galleria mellonella, Grapholita funebrana, Grapholita molesta, Heliothis armigera, Heliothis virescens, Heliothis zea, Hellula undalis, Hibernia defoliaria, Hyphantria cunea, Hyponomeuta malinellus, Keifferia lycopersicella, Lambdina fiscellaria, Laphygma exigua, Leucoptera coffeella, Leucopfera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flamea, Pectinophora gossypiella, Peridroma saucia, Phalera bucephala, Phthorimaea operculella, Phyllocnistis citrella, Pieris brassicae, Plathypena scarbra, Plutella xylostella, Pseudoplusia includens, Phyacionia frustrana, Scrobipalpula absoluta, Sitotroga cerelella, Sparganothis pilleriana, Spodoptera frugiperda, Spodoptera littoralis, Spodoptera litura, Thaumatopoea pityocampa, Tortrix viridana, Trichoplusia ni, Zeiraphera canadensis.

Aus der Ordnung der Käfer (Coleoptera) beispielsweise Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Onlema oryzae, Ortiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus, Sitophilus granaria.

Aus der Ordnung der Zweiflügler (Diptera) beispielsweise Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossia morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea, Tipula paludosa.

Aus der Ordnung der Thripse (Thysanoptera) beispielsweise Frankliniella fusca, Frankliniella occidentalis, Frankliniella tritici, Scirtothrips citri, Thrips oryzae, Thrips palmi, Thrips tabaci.

Aus der Ordnung der Hautflügler (Hymenoptera) beispielsweise Athalia rosae, Atta cephalotes, Atta sexdens, Atta texana, Hoplocampa minuta, Hoplocampa testudinea, Monomorium pharaonis, Solenopsis geminata, Solenopsis invicta.

Aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euchistus impictiventris, Leptoglossus phyllopus, Lygus lineolaris, Lygus pratensis, Nezara viridula, Piesma quadrata, Solubea insularis, Thyanta perditor.

Aus der Ordnung der Pflanzensauger (Homoptera) beispielsweise Acyrthosiphon onobrychis, Adelges laricis, Aphidula nasturtii, Aphis fabae, Aphis pomi, Aphis sambuci, Brachycaudus cardui, Brevicoryne brassicae, Cerosipha gossypii, Dreyfusia nordmannianae, Dreyfusia piceae, Dyasphis radicola, Dysaulacorthum pseudosolani, Empoasca fabae, Macrosiphum avenae, Macrosiphum euphorbiae, Macrosiphon rosae, Megoura viciae, Metopolophium dirhodum, Myzodes persicae, Myzus cerasi, Nilaparvata lugens, Pemphigus bursarius, Perkinsiella saccharicida, Phorodon humuli, Psylla mali, Psylla piri, Rhopalomyzus ascalonicus, Rhopalosiphum maidis, Sappaphis mala, Sappaphis mali, Schizaphis graminum, Schizoneura lanuginosa, Trialeurodes vaporariorum, Viteus vitifolii.

Aus der Ordnung der Termiten (Isoptera) beispielsweise Calotermes flavicollis, Leucotermes flavipes, Reticulitermes lucifugus, Termes natalensis.

Aus der Ordnung der Geradflügler (Orthoptera) beispielsweise Acheta domestica, Blatta orientalis, Blattella germanica, Forficula auricularia, Gryllotalpa gryllotalpa, Locusta migratoria, Melanoplus birittatus, Melanoplus femur-rubrum, Melanoplus mexicanus, Melanoplus sanguinipes, Melanoplus spretus, Nomadacris septemfasciata, Periplaneta americana, Schistocerca americana, Schistocerca peregrina, Stauronotus maroccanus, Tachycines asynamorus.

Aus der Klasse der Arachnoidea beispielsweise Spinnentiere (Acarina) wie Amblyomma americanum, Amglyomma variegatum, Argas persicus, Boophilus annulatus, Boophilus decoloratus, Boophilus microplus, Brevipalpus phoenicis, Bryobia praetiosa, Dermacentor silvarum, Eotetranychus carpini, Eriophyes sheldoni, Hyalomma truncatum, Ixodes ricinus, Ixodes rubicundus, Ornithodorus moubata, Otobins megnini, Paratetranychus pilosus, Permanyssus gallinae, Phyllocaptrata oleivora, Polyphagotarsonemus latus, Psoroptes ovis, Rhipicephalus appendiculatus, Rhipicephalus evertsi, Saccoptes scabiei, Tetranychus cinnabarinus, Tetranychus kanzawai, Tetranychus pacificus, Tetranychus telarius, Tetranychus urticae.

Aus der Klasse der Nematoden beispielsweise Wurzelgallennematoden, z.B. Meloidogyne hapla, Meloidogyne incognita, Meloidogyne javanica, Zysten bildende Nematoden, z.B. Globodera rostochiensis, Heterodera avenae, Heterodera glycinae, Heterodera schatii, Hetrodera triflolii, Stock- und Blattälchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Pratylenchus curvitatus, Pratylenchus goodeyi.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin-Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Die Formulierungen enthalten im allgemeinen zwischen 0,01 und 95 Gew.%, vorzugsweise zwischen 0,1 und 90 Gew.% des Wirkstoffs. Die Wirkstoffe werden dabei in einer Reinheit von 90 % bis 100 %, vorzugsweise 95 % bis 100 % (nach NMR-Spektrum) eingesetzt.

Beispiele für Formulierungen sind:

I. 5 Gew.-Teile der Verbindung Nr. 1 werden mit 95 Gew.-Teilen feinteiligem Kaolin innig vermischt. Man erhält auf diese Weise ein Stäubemittel, das 5 Gew.% des Wirkstoffs enthält.

II. 30 Gew.-Teile der Verbindung Nr. 1 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit (Wirkstoffgehalt 23 Gew.%).

III. 10 Gew.-Teile der Verbindung Nr. 1 werden in einer Mischung gelöst, die aus 90 Gew.-Teilen xylol, 6 Gew.-Teilen des Anlagerungsproduktes von 8 bis 10 Mol Ethylenoxid an 1 Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht (Wirkstoffgehalt 9 Gew.%).

IV. 20 Gew.-Teile der Verbindung Nr. 1 werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5 Gew.-Teilen des Anlagerungsproduktes von 40 Mol Ethylenoxid an 1 Mol Ricinusöl besteht (Wirkstoffgehalt 16 Gew.%).

V. 80 Gew.-Teile der Verbindung Nr. 1 werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen (Wirkstoffgehalt 80 Gew.%).

VI. Man vermischt 90 Gew.-Teile der Verbindung Nr. 1 mit 10 Gew.-Teilen N-Methyl-$\alpha$-pyrrolidon und erhält eine Lösung, die zur Anwendung in Form kleinster Tropfen geeignet ist (Wirkstoffgehalt 90 Gew.%).

VII. 20 Gew.-Teile der Verbindung Nr. 1 werden in einer Mischung gelöst, die aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.% des Wirkstoffs enthält.

VIII. 20 Gew.-Teile des Wirkstoffs Nr. 1 werden mit 3 Gew.-Teilen des Natriumsalzes der Diisobutylnaphthalin-$\alpha$-sulfonsäure, 17 Gew.-Teilen des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20 000 Gew.-Teilen Wasser erhält man eine Spritzbrühe, die 0,1 Gew.% des Wirkstoffs enthält.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel,

Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Wirkstoffkonzentrationen in den anwendungsfertigen Zubereitungen können in größeren Bereichen variiert werden.

Im allgemeinen liegen sie zwischen 0,0001 und 10 %, vorzugsweise zwischen 0,01 und 1 %.

Die Wirkstoffe können auch mit gutem Erfolg im Ultra-Low-Volume-Verfahren (ULV) verwendet werden, wobei es möglich ist, Formulierungen mit mehr als 95 Gew.% Wirkstoff oder sogar den Wirkstoff ohne Zusätze auszubringen.

Die Aufwandmenge an Wirkstoff beträgt unter Freilandbedingungen 0,01 bis 10, vorzugsweise 0,05 bis 5 kg/ha.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Synthesebeispiele

Die im nachstehenden Synthesebeispiel wiedergegebene Vorschrift wurde unter entsprechender Abwandlung der Ausgangsverbindungen zur Gewinnung weiterer Verbindungen IA benutzt. Die so erhaltenen Verbindungen sind in der vorstehenden Tabelle aufgeführt.

Beispiel 1

2,2'-Thienyl-benzothiophen

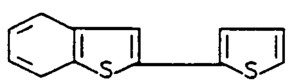

a) 2-Brom-benzothiophen:
In 940 ml absolutem Diethylether werden 94,0 g (0,7 mol) Benzothiophen bei 20-30 °C vorgelegt und innerhalb von 2 Stunden 332 g (0,77 mol) einer 15 %igen n-Butyl-lithiumlösung in n-Hexan zugetropft. Nach einer Stunde werden weiterhin bei 0 °C 127 g (0,799 mol) Brom zugetropft und 2 Stunden nachgerührt. Nach üblicher Aufarbeitung wird zuletzt bei 1-1,2 Torr und 84-86 °C 2-Brom-benzothiophen destilliert.
Ausbeute: 240 g (76 %).
b) 2,2'-Thienyl-benzothiophen
Eine Grignardlösung aus 9,64 g (0,059 mol) 2-Bromthiophen und 1,42 g (0,059 mol) Magnesium in 30 ml Diethylether wird bei 25 °C zu 10 g 2-Brombenzothiophen (0,047 mol) und 0,1 g DPPP-Nickel-Katalysator getropft. Nach 5 Stunden Rühren bei 25 °C wird wie üblich aufgearbeitet und zuletzt aus Ethylalkohol mit etwas Aktivkohle umkristallisiert.
Fp. 134-135 °C; Ausbeute 6,8 g (67 %).

Anwendungsbeispiele

Die insektizide Wirkung der Verbindungen der allgemeinen Formel IA ließ sich durch folgende Versuche zeigen:
Die Wirkstoffe wurden
a) als 0,1 %ige Lösung in Aceton oder
b) als 10 %ige Emulsion in einem Gemisch aus 70 Gew.% Cyclohexanol, 20 Gew.% Nekanil® LN (Lutensol® AP6, Netzmittel mit Emulgier- und Dispergierwirkung auf der Basis ethoxylierter Alkylphenole) und 10 Gew.% Emulphor® EL (Emulan® EL, Emulgator auf der Basis ethoxylierter Fettalkohole)
aufbereitet und entsprechend der gewünschten Konzentration mit Aceton im Fall von a) bzw. mit Wasser im

Fall von b) verdünnt.

Nach Abschluß der Versuche wurde die jeweils niedrigste Konzentration ermittelt, bei der die Verbindungen im Vergleich zu unbehandelten Kontrollversuchen noch eine 80-100 %ige Hemmung bzw. Mortalität hervorriefen (Wirkschwelle bzw. Minimalkonzentration (mg)).

A. Tetranychus telarius (Rote Spinne), Kontaktwirkung

Stark befallene getopfte Buschbohnen, die das zweite Folgeblattpaar zeigten, wurden mit der wässrigen Wirkstoffaufbereitung tropfnaß gespritzt. Nach 5 Tagen im Gewächshaus wurde der Bekämpfungserfolg mittels Binokular bestimmt. In diesem Test zeigte die Verbindung Beispiel 1 eine Wirkschwelle von 100 ppm.

B. Aedes aegypti (Gelbfiebermücke), Zuchtversuch

30-40 Larven des 3. bis 4. Larvenstadiums werden in 200 ml Wasser bei 23 °C mit der wässrigen Wirkstoffaufbereitung behandelt. Nach 24 h wurde die Mortalitätsrate bestimmt. Danach wurden die Versuche bis zum Schlüpfen der Mücken bei einem unbehandelten (Kontroll-)Versuch bei 25 °C belassen und die Entwicklungshemmung im Vergleich zur unbehandelten Kontrolle bestimmt. In diesem Test zeigte die Verbindung Beispiel 1 eine Wirkschwelle von 0,04 ppm.

**Patentansprüche**
**Patentansprüche für folgende Vertragsstaaten : BE, CH, DE, DK, FR, GB, IT, LI, NL**

**1.** 2,2'-Thienyl-benzothiophene der allgemeinen Formel I,

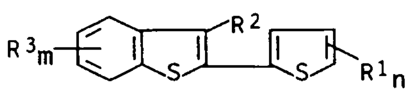

I

in der die Substituenten und die Indizes die folgende Bedeutung haben:

$R^1$ und $R^3$ unabhängig voneinander Halogen, $C_1$-$C_8$-Alkyl, $C_1$-$C_6$-Halogenalkyl, $C_1$-$C_6$-Alkoxy und $C_1$-$C_6$-Halogenalkoxy,

$R^2$ Wasserstoff, Halogen, $C_1$-$C_8$-Alkyl, $C_1$-$C_6$-Halogenalkyl, $C_1$-$C_6$-Alkoxy und $C_1$-$C_6$-Halogenalkoxy,

n 0, 1 oder 2, wobei die Reste verschieden sein können, wenn n für 2 steht,

m 0, 1, 2, 3 oder 4, wobei die Reste verschieden sein können, wenn m für 2, 3 oder 4 steht,

und wobei n und m nicht gleichzeitig 0 bedeuten, wenn $R^2$ Wasserstoff bedeutet.

**2.** 2,2'-Thienyl-benzothiophene der allgemeinen Formel I gemäß Anspruch 1, dadurch gekennzeichnet, daß

$R^1$ und $R^3$ unabhängig voneinander Halogen, $C_1$-$C_6$-Alkyl, $C_1$-$C_4$-Halogenalkyl, $C_1$-$C_4$-Alkoxy und $C_1$-$C_4$-Halogenalkoxy bedeutet,

$R^2$ Wasserstoff, Halogen, $C_1$-$C_6$-Alkyl, $C_1$-$C_4$-Halogenalkyl, $C_1$-$C_4$-Alkoxy und $C_1$-$C_4$-Halogenalkoxy bedeutet,

n 0, 1 oder 2 bedeutet, wobei die Reste verschieden sein können, wenn n für 2 steht,

m 0, 1, 2 oder 3 bedeutet, wobei die Reste verschieden sein können, wenn m für 2 oder 3 steht,

und wobei n und m nicht gleichzeitig 0 bedeuten, wenn $R^2$ Wasserstoff bedeutet.

**3.** 2,2'-Thienyl-benzothiophene der allgemeinen Formel I gemäß Anspruch 1, dadurch gekennzeichnet, daß

$R^1$ und $R^3$ unabhängig voneinander Halogen, $C_1$-$C_4$-Alkyl, $C_1$-$C_2$-Halogenalkyl, $C_1$-$C_2$-Alkoxy und $C_1$-$C_2$-Halogenalkoxy bedeutet,

$R^2$ Wasserstoff, Halogen, $C_1$-$C_4$-Alkyl, $C_1$-$C_2$-Halogenalkyl, $C_1$-$C_2$-Alkoxy und $C_1$-$C_2$-Halogenalkoxy bedeutet,

n 0 oder 1 bedeutet,

m 0, 1 oder 2 bedeutet, wobei die Reste verschieden sein können, wenn m für 2 steht, und wobei n und m nicht gleichzeitig 0 bedeuten, wenn $R^2$ Wasserstoff bedeutet.

**4.** Verfahren zur Herstellung der Verbindungen I gemäß Anspruch 1, dadurch gekennzeichnet, daß man ein 2-Halogen-benzothiophen der allgemeinen Formel II

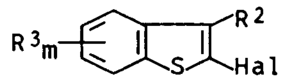

II

in der Hal für ein Halogenatom steht, in an sich bekannter Weise in einem inerten organischen Lösungsmittel in Gegenwart eines Katalysators mit einer Thienyl-Grignard Verbindung der allgemeinen Formel III

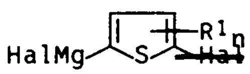

III

in der Hal für ein Halogenatom steht, kuppelt.

**5.** Mittel zu Bekämpfung von Schädlingen, enthaltend eine Verbindung der allgemeinen Formel I gemäß Anspruch 1 und inerte Zusatzstoffe.

**6.** Verfahren zu Bekämpfung von Schädlingen, dadurch gekennzeichnet, daß man die Schädlinge und/oder ihren Lebensraum mit einer wirksamen Menge einer Verbindung der Formel IA

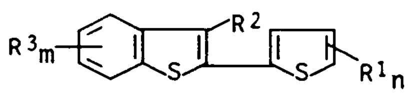

IA

in der die Substituenten und die Indizes die folgende Bedeutung haben:

- $R^1$ und $R^3$ unabhängig voneinander Halogen, $C_1$-$C_8$-Alkyl, $C_1$-$C_6$-Halogenalkyl, $C_1$-$C_6$-Alkoxy und $C_1$-$C_6$-Halogenalkoxy,
- $R^2$ Wasserstoff, Halogen, $C_1$-$C_8$-Alkyl, $C_1$-$C_6$-Halogenalkyl, $C_1$-$C_6$-Alkoxy und $C_1$-$C_6$-Halogenalkoxy,
- n 0, 1 oder 2, wobei die Reste verschieden sein können, wenn n für 2 steht,
- m 0, 1, 2, 3 oder 4, wobei die Reste verschieden sein können, wenn m für 2, 3 oder 4 steht,

behandelt.

**Patentansprüche für folgenden Vertragsstaat : ES**

**1.** Mittel zur Bekämpfung von Schädlingen, enthaltend eine Verbindung der allgemeinen Formel I

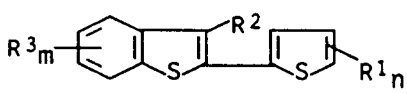

I

in der die Substituenten und die Indizes die folgende Bedeutung haben:

- $R^1$ und $R^3$ unabhängig voneinander Halogen, $C_1$-$C_8$-Alkyl, $C_1$-$C_6$-Halogenalkyl, $C_1$-$C_6$-Alkoxy und $C_1$-$C_6$-Halogenalkoxy,
- $R^2$ Wasserstoff, Halogen, $C_1$-$C_8$-Alkyl, $C_1$-$C_6$-Halogenalkyl, $C_1$-$C_6$-Alkoxy und $C_1$-$C_6$-Halogenalkoxy,
- n 0, 1 oder 2, wobei die Reste verschieden sein können, wenn n für 2 steht,

m 0, 1, 2, 3 oder 4, wobei die Reste verschieden sein können, wenn m für 2, 3 oder 4 steht,

und wobei n und m nicht gleichzeitig 0 bedeuten, wenn $R^2$ Wasserstoff bedeutet und inerte Zusatzstoffe.

**2.** Mittel zur Bekämpfung von Schädlingen nach Anspruch 1, enthaltend 2,2'-Thienyl-benzothiophene der allgemeinen Formel I gemäß Anspruch 1, wobei

$R^1$ und $R^3$ unabhängig voneinander Halogen, $C_1$-$C_6$-Alkyl, $C_1$-$C_4$-Halogenalkyl, $C_1$-$C_4$-Alkoxy und $C_1$-$C_4$-Halogenalkoxy bedeutet,

$R^2$ Wasserstoff, Halogen, $C_1$-$C_6$-Alkyl, $C_1$-$C_4$-Halogenalkyl, $C_1$-$C_4$-Alkoxy und $C_1$-$C_4$-Halogenalkoxy bedeutet,

n 0, 1 oder 2 bedeutet, wobei die Reste verschieden sein können, wenn n für 2 steht,

m 0, 1, 2 oder 3 bedeutet, wobei die Reste verschieden sein können, wenn m für 2 oder 3 steht,

und wobei n und m nicht gleichzeitig 0 bedeuten, wenn $R^2$ Wasserstoff bedeutet.

**3.** Mittel zur Bekämpfung von Schädlingen nach Anspruch 1, enthaltend 2,2'-Thienyl-benzothiophene der allgemeinen Formel I gemäß Anspruch 1, wobei

$R^1$ und $R^3$ unabhängig voneinander Halogen, $C_1$-$C_4$-Alkyl, $C_1$-$C_2$-Halogenalkyl, $C_1$-$C_2$-Alkoxy und $C_1$-$C_2$-Halogenalkoxy bedeutet,

$R^2$ Wasserstoff, Halogen, $C_1$-$C_4$-Alkyl, $C_1$-$C_2$-Halogenalkyl, $C_1$-$C_2$-Alkoxy und $C_1$-$C_2$-Halogenalkoxy bedeutet,

n 0 oder 1 bedeutet,

m 0, 1 oder 2 bedeutet, wobei die Reste verschieden sein können, wenn m für 2 steht,

und wobei n und m nicht gleichzeitig 0 bedeuten, wenn $R^2$ Wasserstoff bedeutet.

**4.** Verfahren zur Herstellung der Verbindungen I gemäß Anspruch 1, dadurch gekennzeichnet, daß man ein 2-Halogen-benzothiophen der allgemeinen Formel II

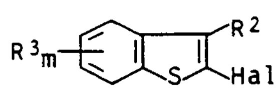

II

in der Hal für ein Halogenatom steht, in an sich bekannter Weise in einem inerten organischen Lösungsmittel in Gegenwart eines Katalysators mit einer Thienyl-Grignard Verbindung der allgemeinen Formel III

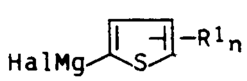

III

in der Hal für ein Halogenatom steht, kuppelt.

**5.** Verfahren zu Bekämpfung von Schädlingen, dadurch gekennzeichnet, daß man die Schädlinge und/oder ihren Lebensraum mit einer wirksamen Menge einer Verbindung der Formel IA

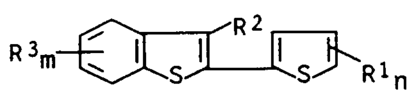

IA

in der die Substituenten und die Indizes die folgende Bedeutung haben:

$R^1$ und $R^3$ unabhängig voneinander Halogen, $C_1$-$C_8$-Alkyl, $C_1$-$C_6$-Halogenalkyl, $C_1$-$C_6$-Alkoxy und $C_1$-$C_6$-Halogenalkoxy,

$R^2$ Wasserstoff, Halogen, $C_1$-$C_8$-Alkyl, $C_1$-$C_6$-Halogenalkyl, $C_1$-$C_6$-Alkoxy und $C_1$-$C_6$-

| | |
|---|---|
| | Halogenalkoxy, |
| n | 0, 1 oder 2, wobei die Reste verschieden sein können, wenn n für 2 steht, |
| m | 0, 1, 2, 3 oder 4, wobei die Reste verschieden sein können, wenn m für 2, 3 oder 4 steht, |

behandelt.

## Claims

**Claims for the following Contracting States : BE, CH, DE, DK, FR, GB, IT, LI, NL**

1. A 2,2'-thienylbenzothiophene of the formula I

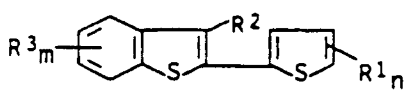

I

where $R^1$ and $R^3$ independently of one another are each halogen, $C_1$-$C_8$-alkyl, $C_1$-$C_6$-haloalkyl, $C_1$-$C_6$-alkoxy or $C_1$-$C_6$-haloalkoxy, $R^2$ is hydrogen, halogen, $C_1$-$C_8$-alkyl, $C_1$-$C_6$-haloalkyl, $C_1$-$C_6$-alkoxy or $C_1$-$C_6$-haloalkoxy, n is 0, 1 or 2, and the radicals may be different when n is 2, m is 0, 1, 2, 3 or 4, and the radicals may be different when m is 2, 3 or 4, and n and m are not simultaneously 0 when $R^2$ is hydrogen.

2. A 2,2'-thienylbensothiophene of the formula I as claimed in claim 1, wherein $R^1$ and $R^3$ independently of one another are each halogen, $C_1$-$C_6$-alkyl, $C_1$-$C_4$-haloalkyl, $C_1$-$C_4$-alkoxy or $C_1$-$C_4$-haloalkoxy, $R^2$ is hydrogen, halogen, $C_1$-$C_6$-alkyl, $C_1$-$C_4$-haloalkyl, $C_1$-$C_4$-alkoxy or $C_1$-$C_4$-haloalkoxy, n is 0, 1 or 2, and the radicals may be different when n is 2, m is 0, 1, 2 or 3, and the radicals may be different when m is 2 or 3, and n and m are not simultaneously 0 when $R^2$ is hydrogen.

3. A 2,2'-thienylbenzothiophene of the formula I as claimed in claim 1, wherein $R^1$ and $R^3$ independently of one another are each halogen, $C_1$-$C_4$-alkyl, $C_1$- or $C_2$-haloalkyl, $C_1$- or $C_2$-alkoxy or $C_1$- or $C_2$-haloalkoxy, $R^2$ is hydrogen, halogen, $C_1$-$C_4$-alkyl, $C_1$- or $C_2$-haloalkyl, $C_1$- or $C_2$-alkoxy or $C_1$- or $C_2$-haloalkoxy, n is 0 or 1, m is 0, 1 or 2, and the radicals may be different when m is 2, and n and m are not simultaneously 0 when $R^2$ is hydrogen.

4. A process for the preparation of a compound I as claimed in claim 1, wherein a 2-halobenzothiophene of the formula II

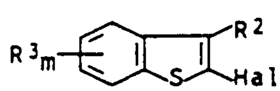

II

where Hal is halogen, is coupled with a thienyl-Grignard compound of the formula III

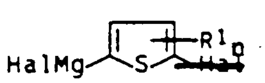

III

where Hal is halogen, in a conventional manner, in an inert organic solvent in the presence of a catalyst.

5. A pesticide containing a compound of the formula I as claimed in claim 1 and inert additives.

6. A method for controlling pests, wherein the pests or their habitat are treated with an effective amount of a compound of the formula IA

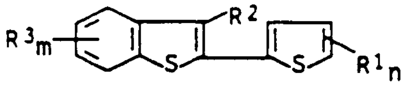

IA

where $R^1$ and $R^3$ independently of one another are each halogen, $C_1$-$C_8$-alkyl, $C_1$-$C_6$-haloalkyl, $C_1$-$C_6$-alkoxy or $C_1$-$C_6$-haloalkoxy, $R^2$ is hydrogen, halogen, $C_1$-$C_8$-alkyl, $C_1$-$C_6$-haloalkyl, $C_1$-$C_6$-alkoxy or $C_1$-$C_6$-haloalkoxy, n is 0, 1 or 2, and the radicals may be different when n is 2 and m is 0, 1, 2, 3 or 4, and the radicals may be different when m is 2, 3 or 4.

**Claims for the following Contracting State : ES**

1. A pesticide containing a compound of the formula I

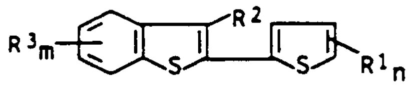

I

where $R^1$ and $R^3$ independently of one another are each halogen, $C_1$-$C_8$-alkyl, $C_1$-$C_6$-haloalkyl, $C_1$-$C_6$-alkoxy or $C_1$-$C_6$-haloalkoxy, $R^2$ is hydrogen, halogen, $C_1$-$C_8$-alkyl, $C_1$-$C_6$-haloalkyl, $C_1$-$C_6$-alkoxy or $C_1$-$C_6$-haloalkoxy, n is 0, 1 or 2, and the radicals may be different when n is 2, m is 0, 1, 2, 3 or 4, and the radicals may be different when m is 2, 3 or 4, and n and m are not simultaneously 0 when $R^2$ is hydrogen, and inert additives.

2. A pesticide as claimed in claim 1, containing a 2,2'-thienylbenzothiophene of the formula I as claimed in claim 1, wherein $R^1$ and $R^3$ independently of one another are each halogen, $C_1$-$C_6$-alkyl, $C_1$-$C_4$-haloalkyl, $C_1$-$C_4$-alkoxy or $C_1$-$C_4$-haloalkoxy, $R^2$ is hydrogen, halogen, $C_1$-$C_6$-alkyl, $C_1$-$C_4$-haloalkyl, $C_1$-$C_4$-alkoxy or $C_1$-$C_4$-haloalkoxy, n is 0, 1 or 2, and the radicals may be different when n is 2, m is 0, 1, 2 or 3, and the radicals may be different when m is 2 or 3, and n and m are not simultaneously 0 when $R^2$ is hydrogen.

3. A pesticide as claimed in claim 1, containing a 2,2'-thienylbenzothiophene of the formula I as claimed in claim 1, wherein $R^1$ and $R^3$ independently of one another are each halogen, $C_1$-$C_4$-alkyl, $C_1$- or $C_2$-haloalkyl, $C_1$- or $C_2$-alkoxy or $C_1$- or $C_2$-haloalkoxy, $R^2$ is hydrogen, halogen, $C_1$-$C_4$-alkyl, $C_1$- or $C_2$-haloalkyl, $C_1$- or $C_2$-alkoxy or $C_1$- or $C_2$-haloalkoxy, n is 0 or 1, m is 0, 1 or 2, and the radicals may be different when m is 2, and n and m are not simultaneously 0 when $R^2$ is hydrogen.

4. A process for the preparation of a compound I as claimed in claim 1, wherein a 2-halobenzothiophene of the formula II

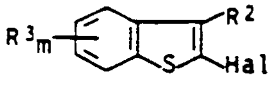

II

where Hal is halogen, is coupled with a thienyl-Grignard compound of the formula III

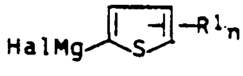

III

where Hal is halogen, in a conventional manner, in an inert organic solvent in the presence of a catalyst.

5. A method for controlling pests, wherein the pests or their habitat are treated with an effective amount of a compound of the formula IA

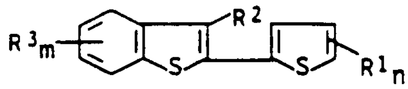

IA

where $R^1$ and $R^3$ independently of one another are each halogen, $C_1$-$C_8$-alkyl, $C_1$-$C_6$-haloalkyl, $C_1$-$C_6$-alkoxy or $C_1$-$C_6$-haloalkoxy, $R^2$ is hydrogen, halogen, $C_1$-$C_8$-alkyl, $C_1$-$C_6$-haloalkyl, $C_1$-$C_6$-alkoxy or $C_1$-$C_6$-haloalkoxy, n is 0, 1 or 2, and the radicals may be different when n is 2 and m is 0, 1, 2, 3 or 4, and the radicals may be different when m is 2, 3 or 4.

**Revendications**

**Revendications pour les Etats contractants suivants : BE, CH, DE, DK, FR, GB, IT, LI, NL**

1. 2,2'-thiényl-benzothiophene de la formule générale I

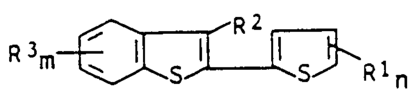

I

dans laquelle les substituants et les indices ont la signification suivante :
$R^1$ et $R^3$, indépendant l'un de l'autre, halogène, alkyle en C1-C8, halogénalkyle en C1-C6, alcoxy en C1-C6 et halogénalcoxy en C1-C6,
$R^2$, hydrogène, halogène, alkyle en C1-C8, halogénalkyle en C1-C6, alcoxy en C1-C6, halogénalcoxy en C1-C6,
n, 0, 1 ou 2, les restes pouvant être différents quand n est mis pour 2,
m, 0, 1, 2, 3 ou 4, les restes pouvant être différents quand m est mis pour 2, 3 ou 4
et n et m ne représentant pas simultanément 0 quand $R^2$ est mis pour hydrogène.

2. 2,2'-thiényl-benzothiophène de la formule générale I selon la revendication 1, caractérisé par le fait que
$R^1$ et $R^3$, indépendant l'un de l'autre représentent halogène, alkyle en C1-C6, halogénalkyle en C1-C4, alcoxy en C1-C4 et halogénalcoxy en C1-C4,
$R^2$ représente hydrogène, halogène, alkyle en C1-C6, halogénalkyle en C1-C4, alcoxy en C1-C4 et halogénalcoxy en C1-C4,
n représente 0, 1 ou 2, les restes pouvant être différents quand n est mis pour 2,
m représente 0, 1, 2 ou 3, les restes pouvant être différents quand m est mis pour 2 ou 3
et n et m ne représentant pas simultanément 0 quand $R^2$ est mis pour hydrogène.

3. 2,2'thiényl-benzothiophène de la formule générale I selon la revendication 1 caractérisé par le fait que
$R^1$ et $R^3$ indépendant l'un de l'autre représentent halogène, alkyle en C1-C4, halogénalkyle en C1-C2, alcoxy en C1-C2 et halogénalcoxy en C1-C2,
$R^2$ représente hydrogène, alkyle en C1-C4, halogénalkyle en C1-C4, alcoxy en C1-C2 et halogénalcoxy en C1-C2,
n représente 0 ou 1,
m représente 0, 1 ou 2, les restes pouvant être différents quand m est mis pour 2
et n et m ne représentant pas simultanément 0 quand $R^2$ est mis pour hydrogène.

4. Procédé de préparation des composés I selon la revendication 1 , caractérisé par le fait que l'on accouple un 2-halogéno-benzathiophène de la formule générale II

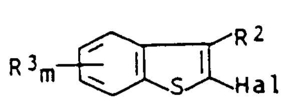

II

dans laquelle Hal est mis pour un atome d'halogène, de manière connue en soi, dans un solvant organique inerte, en présence d'un catalyseur avec un dérivé de Grignard de thiényle de la formule générale III

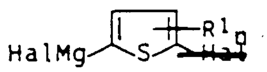

III

dans laquelle Hal est mis pour un atome d'halogène.

**5.** Moyen de lutte contre les parasites, contenant un composé de la formule générale I selon la revendication 1 et des additifs inertes.

**6.** Procédé de lutte contre les parasites, caractérisé par le fait que l'on traite les parasites et/ou leur biotope avec une quantité efficace d'un composé de la formule IA

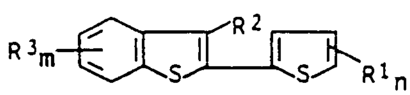

IA

dans laquelle les substituants et les indices ont la signification suivante :
$R^1$ et $R^3$, indépendant l'un de l'autre, halogène, alkyle en C1-C8, halogénalkyle en C1-C6, alcoxy en C1-C6 et halogénalcoxy en C1-C6,
$R^2$, hydrogène, halogène, alkyle en C1-C8, halogénalkyle en C1-C6, alcoxy en C1-C6 et halogénalcoxy en C1-C6,
n, 0, 1 ou 2, les restes pouvant être différents quand n est mis pour 2,
m, 0, 1, 2, 3 ou 4, les restes pouvant être différents quand m est mis pour 2, 3 ou 4.

**Revendications pour l'Etat contractant suivant : ES**

**1.** Moyen de lutte contre les pesticides contenant un composé de la formule générale I

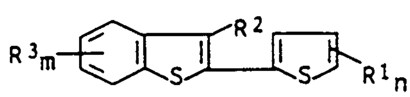

I

dans laquelle les substituants et les indices ont la signification suivante :
$R^1$ et $R^3$, indépendant l'un de l'autre, halogène, alkyle en C1-C8, halogénalkyle en C1-C6, alcoxy en C1-C6 et halogénalcoxy en C1-C6,
$R^2$, hydrogène, halogène, alkyle en C1-C8, halogénalkyle en C1-C6, alcoxy en C1-C6, halogénalcoxy en C1-C6,
n, 0, 1 ou 2, les restes pouvant être différents quand n est mis pour 2,
m, 0, 1, 2, 3 ou 4, les restes pouvant être différents quand m est mis pour 2, 3 ou 4
et n et m ne représentant pas simultanément 0 quand $R^2$ est mis pour hydrogène.

**2.** Moyen de lutte contre les pesticides selon la revendication 1, contenant 2,2'-thiényl-benzothiophène de la formule générale I selon la revendication 1, caractérisé par le fait que
$R^1$ et $R^3$ indépendant l'un de l'autre représentent halogène, alkyle en C1-C6, halogénalkyle en C1-C4, alcoxy en C1-C4 et halogénalcoxy en C1-C4,
$R^2$ représente hydrogène, halogène, alkyle en C1-C6, halogénalkyle en C1-C4, alcoxy en C1-C4 et halogénalcoxy en C1-C4,
n représente 0, 1 ou 2, les restes pouvant être différents quand n est mis pour 2,

m représente 0, 1, 2 ou 3, les restes pouvant être différents quand m est mis pour 2 ou 3 et n et m ne représentant pas simultanément 0 quand $R^2$ est mis pour hydrogène.

**3.** Moyen de lutte contre les pesticides selon la revendication 1, contenant 2,2'-thiényl-benzothiophène de la formule générale I selon la revendication 1, caractérisé par le fait que
$R^1$ et $R^3$ indépendant l'un de l'autre représentent halogène, alkyle en C1-C4, halogénalkyle en C1-C2, alcoxy en C1-C2 et halogénalcoxy en C1-C2,
$R^2$ représente hydrogène, alkyle en C1-C4, halogénalkyle en C1-C4, alcoxy en C1-C2 et halogénalcoxy en C1-C2,
n représente 0 ou 1,
m représente 0, 1 ou 2, les restes pouvant être différents quand m est mis pour 2
et n et m ne représentent pas simultanément 0 quand $R^2$ est mis pour hydrogène.

**4.** Procédé de préparation des composés I selon la revendication 1, caractérisé par le fait que l'on accouple un 2-halogéno-benzathiophène de la formule générale II

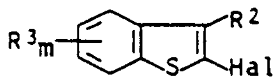

II

dans laquelle Hal est mis pour un atome d'halogène, de manière connue en soi, dans un solvant organique inerte, en présence d'un catalyseur avec un dérivé de Grignard de thiényle de la formule générale III

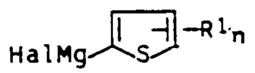

III

dans laquelle Hal est mis pour un atome d'halogène.

**5.** Procédé de lutte contre les parasites, caractérisé par le fait que l'on traite les parasites et/ou leur biotope avec une quantité efficace d'un composé de la formule IA

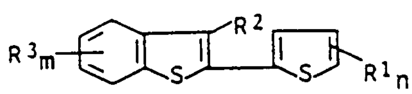

IA

dans laquelle les substituants et les indices ont la signification suivante :
$R^1$ et $R^3$, indépendant l'un de l'autre, halogène, alkyle en C1-C8, halogénalkyle en C1-C6, alcoxy en C1-C6 et halogénalcoxy en C1-C6,
$R^2$, hydrogène, halogène, alkyle en C1-C8, halogénalkyle en C1-C6, alcoxy en C1-C6 et halogénalcoxy en C1-C6,
n, 0, 1 ou 2, les restes pouvant être différents quand n est mis pour 2,
m, 0, 1, 2, 3 ou 4, les restes pouvant être différents quand m est mis pour 2, 3 ou 4.